(19) Europäisches Patentamt
European Patent Office
Office européen des brevets

(11) Publication number: **0 162 330 B1**

(12) **EUROPEAN PATENT SPECIFICATION**

(45) Date of publication of patent specification: **10.03.93**

(51) Int. Cl.5: **A61K 31/705**, A61K 31/57, A61K 31/58

(21) Application number: **85105085.6**

(22) Date of filing: **26.04.85**

(54) **Use of sterolins and spiroketalins in the production of medicaments for the treatment of psoriasis.**

(30) Priority: **30.04.84 DE 3416112**

(43) Date of publication of application:
**27.11.85 Bulletin 85/48**

(45) Publication of the grant of the patent:
**10.03.93 Bulletin 93/10**

(84) Designated Contracting States:
**AT BE CH DE FR GB IT LI LU NL SE**

(56) References cited:
**EP-A- 0 007 474**
**DE-A- 2 113 215**
**DE-A- 2 759 171**
**GB-A- 2 024 624**

(73) Proprietor: **Roecar Holdings (Netherlands Antilles) N.V.**
**Rokin 84**
**Amsterdam - C(NL)**

(72) Inventor: **Walker, Hans, Dr.**
**Hessenring 29**
**W-3440 Eschwege(DE)**
Inventor: **Pegel, K. H., Prof.**
**Dept. of Chemistry King George V Ave.**
**Durban 4001(ZA)**

(74) Representative: **Siewers, Gescha, Dr. et al**
**Rechtsanwälte Dr. Harmsen, Dr. Utescher**
**Dipl.-Chem. Harmsen, Bartholatus Dr.**
**Schaeffer, Dr. Fricke, Wolter Patentanwalt Dr.**
**Siewers Adenauerallee 28**
**W-2000 Hamburg 1 (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to the use of sterolins and/or spiroketalins in the production of the medicaments for the treatment of psoriasis.

Only recently has it become known and accepted that arachidonic acid is one of the key precursor substances in the complex pathway of the body's metabolism. Although it has been known for some time that through the action of the cyclooxygenase and including ring- and endoperoxide reactions the different prostaglandines, thromboxanes and prostacyclines are formed from arachidonic acid, it has been found only recently that arachidonic acid acts also as the precursor for the so called leukotrienes. The most important difference in comparison to the synthesis of the prostaglandines is that the substrate arachidonic acid does not interact with cyclooxygenase but with lipoxygenase.

Besides the classic allergy factors, such as histamine, serotonin and kinins, leukotrienes are gaining more and more importance as mediators in wide variety of allergic disorders. Compounds collectively known as leukotrienes, are derivatives of 5-hydroxy-7,9,11,14-eicosatetraenoic acid which are linked in position 6 via a thioether bridge to glutathion or which are hydroxylated in position 12 via addition of a molecule of water.

The importance and the mode of action of the leukotrienes in the body are not yet fully understood, but it appears that these compounds play an important role in the manifestation of several allergic reactions. It is known that histamin and leukotrienes are present in the so called basophilic granulocytes and that during allergic reactions immunglobulines of the type E attach to the outer cell membrane of the granulocytes thus increasing their permeability, liberating stored free histamin and activated leukotrienes into the blood and tissue. It is therefore suspected at present that not only biogenic amines and kinins but also leukotrienes may play an important role during allergic reactions of the spontaneous type. Diseases of this kind are for example, asthma and some atopic skin disorders. Excessive liberation of these highly active compounds probably contributes to a large extent to the so called anaphylactic shock syndrome.

At present, it is also not clear by which mechanism(s) the leukotrienes may play an active role in so called autoimmune diseases such as for example rheumatoid arthritides, lupus erythematosus, certain anaemias, for instance leuko-, thrombo- or panzytopenias, certain hepatitides, myasthenia gravis, and possibly a large number of other disorders falling into this group. It was found by various workers in the field that during these diseases the concentration of leukotrienes in blood and/or in tissues and fluids increased. The relationship between leukotrienes and the genesis of psoriasis has not yet been clarified, but it has been shown that in the exudate of psoriatic areas increased concentration of these compounds can be detected. The mechanism(s) for this observation is poorly understood.

A pure selective regulator or inhibitor of the degenerated leukotriene synthesis is not yet available for clinical use. As non-specific inhibitors corticosteroids and benoxaprofen are used in medication of some of these diseases because these compounds either block the liberation of arachidonic acid or in the case of benoxaprofen the conversion from arachidonic acid to leukotrienes and some of their derivatives. The use of Sterolins and spiroketalins for the treatment of asthma and autoimmune diseases like rheumatoid arthritis had already been described in DE-A-2759171.

Unexpectedly and surprisingly it has now been found that sterolins and spiroketalins are active in the treatment of psoriasis.

The glycosides of phytosterols including cholesterol and tetracyclic triterpenes for example lanosterol and cycloartenol are designated as sterolins. Sterolins or phytosterols are present in varying amounts in most plants and are frequently found in micro organisms. Sterolins can be produced synthetically using the known Koenigs-Knorr synthesis reacting the corresponding aglycones and a C-1 brominated sugar acetate using silver oxide or carbonate or mercury or cadmium compounds as catalyst (see UK Pat. No GB-A-1595247β -sitosterol glucoside). The sterolins correspond to the following general formula.

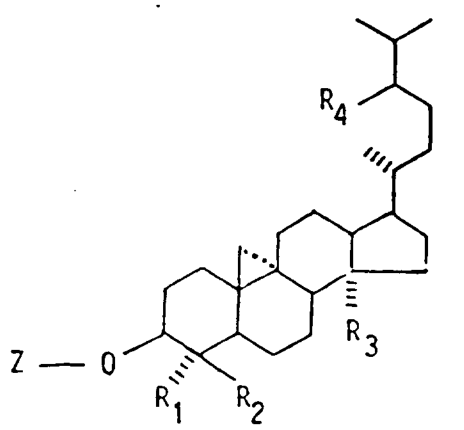

in which R-1, R-2 and R-3 represent hydrogen atoms or methyl group and R-4 a hydrogen atom or a methyl-, ethyl-, methylene- or ethylidene group and that Z represents a sugar group which can be esterified. Double bonds can be present in different positions of the basic skeleton or in the side chain, especially in position 5 if C-5 is not linked to 5-alpha-hydrogen.

Sterolins occur in nature mostly as monoglycosides though some oligoglycosides have been described. The most common sugar is D-glucose but in the naturally occurring compounds mannose, galactose, arabinose and xylose have also been found. The natural sterolins also occur in part in the form of their esters usually with monobasic monocarboxylic acids, mainly as palmitates.

The compounds can be processed into pharmaceutical compositions such as powders, tablets, capsules, sugar coated pills, solutions, emulsions, lotions, solutions for injection or infusion, ointments and creams in a manner known per se. However, it is to be noted, that sterolins and to a lesser extent spiroketalins have only a very limited solubility in water. The compounds, therefore, must be administered in a particle size which is suitable for absorption. Reduction of particle size can be made in the known way by adsorption, enhancement of absorption by addition of carriers and surfactants or milling with or without additives under the proviso that particle sizes are smaller than 100 µm but preferably below 50 µm.

The compounds used according to the invention have a low degree of toxicity and wide therapeutic margins, but they only need to be administered in very small dosages for an effective treatment. The daily dosage range is about 0.01 to 10 mg, in general 0.45 to 1.0 mg are sufficient for prophylactic or maintenance treatment.

The mono- to tri-glycosides of steroidsaponins are designated as spiroketalins which possess in the steroid skeleton of the aglycone moiety a spiroketal group connected to C-16 and C-17. These aglycones can be considered as 5-en-steroid sapogenins or 5-alpha-steroid sapogenins. Typical 5-en-compounds are for example diosgenin, yamogenin and botogenin whereas tigogenin, hecogenin or sisalagenin are belonging to the 5-alpha compounds. In natural saponin material the aglycones are present as glycosides containing mostly three or more mono saccharide groups and these are split hydrolytically by enzymes or the stomach acid into spiroketalins.

The spiroketalsteroidglycosides correspond to the following general formula:

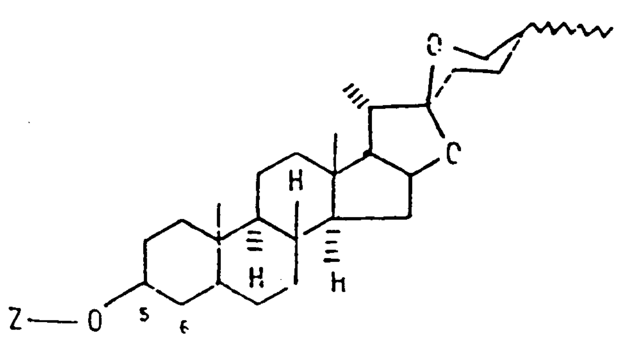

in which a double bond or an alpha-hydrogen can exist in the 5 position and in which Z means a sugar group, which can also be esterified. Spiroketalins can also be synthesized according to the Koenigs-Knorr

synthesis (W Koenigs and E Knorr; Chem. Ber., 1901, 34; 952 or its modifications (C Meystre and K Miescher Helv. Chim. Acta; 1944, 27, 231-236; R B Courow and S Bernstein; Org. Chem.; 1971, 36, 853-870; J J Schneider; Carbohyd. Res.; 1970, 12; 369-389; G Wulff and G Roehle; Angew. Chemie. 1974; 86, 173-187; N Weber; Chem. Phys. Lipids; 1977; 18, 145-148.) or by the orthoester method (N I Ovarova; Carbohyd. Res. 1973; 27; 79-87) by treating 3-$\beta$-hydroxyspiroketalsteroid aglycones with either a C-1 brominated or a 1,2-orthoester mono- or disaccharide acetate in the presence of silver oxide or silver carbonate or other suitable catalysts.

In addition the mono- and disaccharide glycoside of spiroketalins may also be obtained by controlled fermentation or acid hydrolysis procedures of the sugar richer spiroketalsteroidsaponins.

The invention will now be described in more detail in the following examples.

Example 1

Influence upon lipoxygenase.

The tests were performed according to the method devised by Schroer et al, Naunyn-Schmiedeberg's Arch. Pharmak. 313, 69 (1980). According to this method washed human platelets are stimulated with thrombin (0.6 iU/ml). Arachidonic acid released from the platelets upon stimulation is transformed by the 12-lipoxygenase of the platelets into 12-hydro-peroxy-eicosatetraenoic acid (12-HPETE). Diosgeninglucoside in a dosage of 5 $\mu$M/ml produced an inhibition of 13%, at a dosage of 10 $\mu$M/ml an inhibition of 31%, and at 50 uM/ml 60%.

One of the most potent experimental lipoxygenase inhibitor known at present is 5,8,11,14-eicosatetraynoic acid (ETYA) which under the above detailed test conditions at a concentration of 10 $\mu$M/ml produced an inhibition of 97%.

Example 2

In vitro determination of inhibition.

Inhibition of e.g. 12-lipoxygenase was proved in further tests on human platelets or leucocyte preparations.

Washed and resuspended platelets or leucocytes were incubated in one test with a saturated solution of $\beta$-sitosterol $\beta$-D-glucoside (23,5 mg/10ml ethanol) and in a second test with a 10 times oversaturated solution with addition of 0.25 $\mu$Ci arachidonic acid for 5 minutes. The test mixtures were then acidified and extracted with ethyl acetate. Separation of the reaction products was performed by radio-thinlayer-chromatography.

The results are presented in the following TABLE 1. The number of leukocytes in test 1 was 2000 and in test 2, 1600 and the number of platelets in the platelets test 1 was 140 000 and in test 2 it was 240 000.

After incubation with the test substance a significant inhibition of the lipoxygenase products can be shown when using platelets leading to a near complete synthesis inhibition and accumulation of non-metabolized arachidonic acid. As far as leucocytes are concerned, there is a dosage-dependant inhibitory effect of the lipoxygenase whereby the changes are mainly based on 12-hydroxyeicoscatetraenoic acid (12-HETE) and 12-HPETE while lesser changes are observed for HHT.

Example 3

In vivo tests

The effect of $\beta$-Sitosterol-$\beta$-D-glucoside was investigated on antigen-induced SRS-A (leukotriene) mediated bronchoconstriction according to the method of Anderson et al (Br. J. Pharmacol. 78,67-74, 1983). Twenty-eight to thirty-three days after the single ovalbumine dose the guinea pigs were anaesthetised and surgically prepared for intravenous administration of the test compounds and measuring the ventilatory pressure. Prior to the antigen (ovalbumine) challenge the animals were adequately pretreated with indomethacin, pyrilamine and propanolol to eliminate the effect of other interfering mediators released during the anaphylactic reaction. The test substance was administered 1 minute before the ovalbumine challenge.

The vehicle (50% DMSO in water) served as a control. Phenindone was used as the standard reference substance. The results are presented in the TABLE 2.

TABLE 1

| "IN VITRO" LIPOXYGENASE INHIBITION BY $\beta$-SITOSTEROL $\beta$-D-GLUCOSIDE | | |
|---|---|---|
| | | 12 HETE 12 HPETE in % |
| 1. | LEUKOCYTES normal<br>30' saturated<br>120' saturated<br>30' oversaturated | 50.2<br>35.9<br>13.4<br>12.4 |
| 2. | LEUKOCYTES normal<br>30' saturated<br>120' saturated<br>30' oversaturated | 48.0<br>37.0<br>2.7<br>22.7 |
| 1. | PLATELETS normal<br>120' saturated | 39.8<br>3.8 |
| 2. | PLATELETS normal<br>120' saturated | 35.0<br>11.9 |

TABLE 2

"IN VIVO" LIPOXYGENASE INHIBITION BY β-SITOSTEROL β-D-GLUCOSIDE

| AGENT | Dose (mg/kg i.v.) | MEAN CHANGE (AS % OF MAX ±S.E.M.) IN VENTILATION PRESSURE AFTER OVALBUMIN ADMINISTRATION MINUTES AFTER CHALLENGE | | | | | | |
|---|---|---|---|---|---|---|---|---|
| | | 1 | 2 | 3 | 4 | 5 | 7.5 | 10 |
| 50% DMSO | - | 7.9 ±2.7 | 55.6 ±9.7 | 75.3 ±6.5 | 76.8 ±5.5 | 83.1 ±4.2 | 72.8 ±6.1 | 65.4 ±8.0 |
| β-Sitosterol-β-D-glucoside | 1 | 5.8 ±4.5 | 26.8* ±8.2 | 30.0** ±10.6 | 38.0** ±9.4 | 39.5*** ±9.3 | 32.9*** ±7.2 | 36.0* ±7.2 |
| β-Sitosterol-β-D-glucoside | 10 | 1.8 ±2.8 | 31.0 ±9.8 | 41.1* ±10.5 | 50.3* ±9.1 | 52.9** ±7.7 | 47.4** ±5.8 | 37.6** ±4.5 |
| Phenidone | 10 | 3.1 ±1.0 | 22.8* ±7.0 | 43.3** ±8.2 | 50.0* ±9.9 | 42.5** ±8.9 | 40.4** ±8.2 | 34.3* ±6.7 |

* Indicates the significance of difference from vehicle-treated group at the equivalent time after antigen-challenge using Student's "t" test (n=8)

* P 0,05 ** P 0,01 *** P 0,001

## Claims

1. Use of Sterolins and/or Spiroketalins in the production of the medicaments for the treatment of Psoriasis.

## Patentansprüche

1. Verwendung von Sterolinen und/oder Spiroketalinen in der Herstellung von Arzneimitteln für die Behandlung von Psoriasis.

## Revendications

1. Utilisation de sterolines et/ou de spiroketalines en la production de médicaments pour le traitement du psoriasis.